Europäisches Patentamt

**(19)** European Patent Office

Office européen des brevets

**(11)** Numéro de publication : **0 248 173 B1**

**(12)** FASCICULE DE BREVET EUROPEEN

**(45)** Date de publication du fascicule du brevet :
06.03.91 Bulletin 91/10

**(51)** Int. Cl.$^5$ : **A41B 9/00, A41B 9/12**

**(21)** Numéro de dépôt : 87105444.1

**(22)** Date de dépôt : 13.04.87

**(54)** Utilisation comme protection pour l'essayage de vêtements d'un flan jetable.

**(30)** Priorité : 01.05.86 CH 1795/86

**(43)** Date de publication de la demande :
09.12.87 Bulletin 87/50

**(45)** Mention de la délivrance du brevet :
06.03.91 Bulletin 91/10

**(84)** Etats contractants désignés :
AT BE CH DE ES FR GB GR IT LI LU NL SE

**(56)** Documents cités :
FR-A- 2 302 699
FR-A- 2 409 020
FR-A- 2 429 566

**(73)** Titulaire : d'Huissier, Dominique
1, Place des Augustins
CH-1205 Genève (CH)

**(72)** Inventeur : d'Huissier, Dominique
1, Place des Augustins
CH-1205 Genève (CH)

**(74)** Mandataire : Micheli, Michel-Pierre et al
MICHELI & CIE, Rue de Genève 122, Case
Postale 61
CH-1226 Genève-Thonex (CH)

**Description**

Lors de l'essayage de vêtements et notamment de costumes de bain, caleçons de bain, sous-vêtements, slips, culottes ou lingerie fine, etc., on est toujours confronté à un problème d'hygiène et d'esthétique. En effet par mesure d'hygiène, il convient de garder sur soi au moins un slip lors de l'essayage de nouveaux vêtements ce qui fréquemment, notamment lors de l'essayage de costumes de bain ou de lingerie fine, empêche de se rendre compte normalement si la pièce essayée convient parfaitement ou non car le slip que l'on a gardé sur soi modifie la silhouette particulièrement en imprimant des lignes, plis ou bourrelets au travers du vêtement que l'on essaye.

De nombreuses personnes sont ainsi tentées d'essayer un vêtement sans protection directement sur leur peau au détriment de toute mesure d'hygiène, notamment lorsque le vêtement n'est finalement pas acheté. En outre, la protection qu'offre un slip que l'on garde sur soi est toute aléatoire car ce slip peut lui-même ne pas être impeccablement propre et n'est de toute façon pas étanche.

Les documents FR-A-2.302.699 et FR-A-2.409.020 concernent des couches-culottes et culottes pour bébés qui répondent à une toute autre préoccupation et en tous cas ne tiennent aucun compte de l'impératif de ne pas modifier la silhouette du corps de la personne qui les porte.

Le document FR-A-2.409.020 se rapporte plus particulièrement à un procédé de fabrication de couche-culottes formée d'une feuille de matière plastique comportant le long de ses bords latéraux une bande de matière autocollante.

Le document FR-A-2.429.566 concerne des sous-vêtements, des maillots de bain, voire même des vêtements pouvant être mis en place à l'aide de parties adhésives.

Aucun de ces documents ne suggère l'utilisation d'un flan en matière synthétique comme protection lors de l'essayage de vêtements.

La présente invention a pour but de remédier aux inconvénients précités et a pour objet l'utilisation comme protection pour l'essayage de vêtements d'un flan jetable en matière synthétique, souple, étanche, présentant sur ses bords supérieur et inférieur des zones auto-adhérentes.

Le dessin annexé illustre schématiquement et à titre d'exemple plusieurs formes d'exécution d'un dispositif de protection.

La figure 1 est une vue en plan d'une première forme d'exécution du dispositif à plat.

La figure 2 est une vue du dispositif illustré à la figure 1 lors de son utilisation.

Les figures 3 et 4 illustrent en plan deux autres formes d'exécution du dispositif à plat.

La figure 5 est une vue du dispositif illustré à la figure 3 lors de son utilisation.

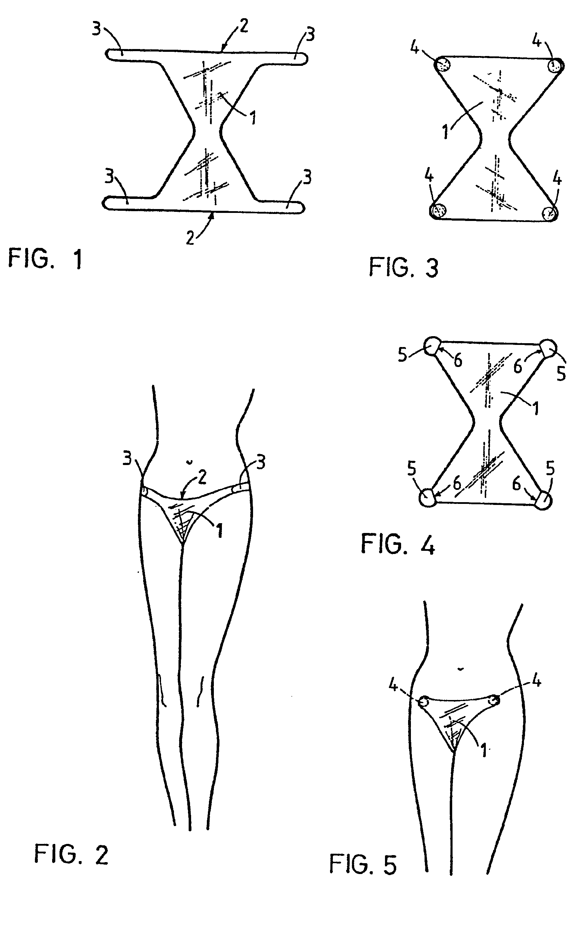

Le dispositif illustré aux figures 1 et 2 est constitué par un flan 1 de feuille d'une matière plastique mince, étanche et souple découpé en forme générale de diabolo dont les bords supérieur et inférieur 2, sont munis de bandelettes ou prolongements 3.

La matière synthétique utilisée pour la réalisation du flan 1 est résistante, étanche, souple, mince, légèrement élastique ou ductile pour, lors de son utilisation, épouser sans pli les formes d'une personne. De préférence cette matière synthétique présente la faculté de coller ou d'adhérer à elle-même lorsqu'elle est repliée sur elle-même. Cette propriété peut provenir de la nature même de la matière utilisée ou d'un traitement de surface que certaines parties des bandelettes 3 auraient subi, par exemple l'enduction d'une substance autocollante.

L'utilisation de ce dispositif de protection est extrêmement simple, il se place entre les jambes, enveloppe le ventre et les fesses de l'usager et se referme autour de la taille, les bandelettes 3 se fixant les unes contre les autres par simple application.

La feuille étant étanche, on obtient une excellente protection. De plus cette feuille étant très mince, de l'ordre du dixième de millimètre ou moins par exemple, souple et légèrement déformable, elle épouse parfaitement le corps de l'usager et ne provoque aucune déformation d'un vêtement qui est enfilé par dessus cette protection pour son essayage.

Le flan est très bon marché de sorte que ce dispositif peut être distribué gratuitement aux usagers aux points de vente de vêtements, des distributeurs automatiques de ces dispositifs peuvent même être prévus.

Il est évident que ces dispositifs peuvent être fabriqués en plusieurs tailles pour satisfaire aux besoins de toute la clientèle.

Enfin, pour que ce dispositif puisse être distribué gratuitement, il peut évidemment servir de support publicitaire, notamment pour des marques de vêtements.

La feuille constituant le flan 1 peut être transparente ou opaque, elle peut également être teintée dans la masse, de préférence couleur chair, afin de ne pas altérer la couleur apparente d'un vêtement que l'on essaye par dessus cette protection.

La seconde forme d'exécution du dispositif illustrée aux figures 3 et 5 comporte également un flan 1 en matière synthétique comme décrit plus haut, présentant la forme générale d'un diabolo. Les coins supérieur et inférieur de ce flan 1 comportent des zones ou pastilles autocollantes recouvertes ou non d'un papier à enlever.

La figure 5 illustre l'utilisation de ce dispositif qui se fixe directement sur la peau de l'utilisateur à l'aide des zones ou pastilles autocollantes 4.

Dans la variante illustrée à la figure 4, les coins du flan 1 sont munis d'oreilles 5 dont une face est autocollante. Lors du stockage des dispositifs, ces oreilles peuvent être repliées suivant une ligne 6 et collées contre le flan 1.

Pour son utilisation, les oreilles 5 sont décollées du flan 1 et dépliées puis servent à maintenir le dispositif en position de service, comme dans le cas de la figure 5, les zones collantes des oreilles 5 étant appliquées directement contre la peau de l'usager.

## Revendications

1. Utilisation comme protection pour l'essayage de vêtements d'un flan jetable en matière synthétique, souple, étanche, présentant sur ses bords supérieur et inférieur des zones auto-adhérentes.

2. Utilisation selon la revendication 1, caractérisée par le fait que la matière synthétique formant le flan présente une épaisseur de l'ordre de 1/10 de mm et qu'elle est légèrement déformable pour épouser étroitement le corps de l'usager sans provoquer de déformation du vêtement essayé.

3. Utilisation selon la revendication 1 ou la revendication 2, caractérisée par le fait que le flan présente la forme générale d'un diabolo et qu'il comporte une indication publicitaire.

4. Utilisation selon la revendication 1 ou la revendication 2, caractérisée par le fait qu'il est teinté couleur chair dans la masse.

5. Utilisation selon l'une des revendications précédentes, caractérisée par le fait que les zones auto-adhérentes sont collées directement sur la peau de l'usager.

## Ansprüche

1. Verwendung eines wegwerfbaren Zuschnittes aus weichem, dichtem Kunststoff als Schutzvorrichtung für das Anprobieren von Kleidungsstücken, wobei der Zuschnitt an seinen oberen und unteren Rändern Selbstklebebereiche aufweist.

2. Verwendung nach Anspruch 1, dadurch gekennzeichnet, daß der den Zuschnitt bildende Kunststoff eine Dicke in der Größenordnung von 1/10 mm hat und leicht verformbar ist, um sich dem Körper des Benützers eng anzupassen, ohne Verformungen des zu probierenden Kleidungsstückes hervorzurufen.

3. Verwendung nach Anspruch 1 oder 2, dadurch gekennzeichnet, daß der Zuschnitt die allgemeine Form eines Diabolo hat und eine Werbenachricht trägt.

4. Verwendung nach Anspruch 1 oder 2, dadurch gekennzeichnet, daß der Zuschnitt in Hautfarbe durchgefärbt ist.

5. Verwendung nach einem der vorhergehenden Ansprüche, dadurch gekennzeichnet, daß die selbstklebenden Bereiche direkt auf die Haut des Anwenders geklebt werden.

## Claims

1. Use as protection for trying on clothing of a thrown away blank made in synthetical material, supple, watertight, presenting on its upper and lower edges auto-adhesiv areas.

2. Use according to claim 1, characterized by the fact that the synthetical material forming the blank has a thickness of about 1/10 mm and that it its slightly deformable to fit closely the body of the wearer without causing deformation of the tried on clothing.

3. Use according to claim 1 or claim 2, characterized by the fact that the blank has the general shape of a hourglass and that it comprises advertisement.

4. Use according to claim 1 or claim 2, characterized by the fact that it is coloured in its mass with a skin colour.

5. Use according to one of the preceeding claims, characterized by the fact taht the auto-adhesiv areas are glued direclty onto the skin of the user.

FIG. 1

FIG. 2

FIG. 3

FIG. 4

FIG. 5